# EUROPÄISCHE PATENTSCHRIFT

(11) **EP 0 739 975 B1**
(45) Veröffentlichungstag und Bekanntmachung des Hinweises auf die Patenterteilung: **11.11.1998**
(21) Anmeldenummer: 96101760.5
(22) Anmeldetag: 07.02.1996
(51) Int. Cl.: C11B 1/10, C07H 15/04

(54) **Verfahren zur Reinigung von Kohlenhydratderivaten mit oberflächenaktiver Wirkung**
Method of purifying carbohydrate derivatives with surface active activity
Procédé pour la purification des dérivés carbohydrés avec une activité surfactante

(30) Priorität: 08.02.1995 DE 19504101
(43) Veröffentlichungstag der Anmeldung: 30.10.1996
(73) Patentinhaber: SKW Trostberg Aktiengesellschaft, 83308 Trostberg (DE)
(72) Erfinder: Heidlas, Jürgen, Dr., D-83308 Trostberg (DE); Cully, Jan, Dr., D-84518 Garching (DE)
(74) Vertreter: Weiss, Wolfgang, Dipl.-Chem. Dr.

(56) Entgegenhaltungen:
- EP-A- 0 591 981
- EP-A- 0 615 694
- EP-A- 0 616 025
- US-A- 2 548 434
- US-A- 2 682 551

## Beschreibung

Die vorliegende Erfindung betrifft ein Verfahren zur Reinigung von Kohlenhydratderivaten mit oberflächenaktiver Wirkung mit Hilfe von verdichteten Gasen.

Im Bereich der preisgünstigen nachwachsenden Rohstoffe haben Kohlenhydrate in der jüngsten Zeit immer größere Bedeutung gerade auch als chemische Grundstoffe erlangt. Ein herausragendes Beispiel hierfür sind synthetische Kohlenhydrat-Verbindungen mit oberflächenaktiver Wirkung, sog. Surfactants, bei denen eine hydrophile Polyhydroxystruktur von meist mono-, di- oder oligosaccharidischen Kohlenhydraten mit der lipophilen Struktur von hydrophoben Kohlenwasserstoffketten, z.B. längeren Alkyl- oder Alkylenketten, im Molekül kombiniert sind. Obwohl chemisch mehrere Möglichkeiten zur Verknüpfung von Kohlenhydraten mit Kohlenwasserstoffketten bestehen, wie z.B. über Ester-, Ether-, Amid- oder Aminofunktionen, haben sich im wesentlichen Kohlenhydratester (Zuckerester) und Alkylglycoside (1-0-Zuckerether) oder deren Kombinationen, z.B. veresterte Alkylglycoside, in der industriellen Produktion durchgesetzt. Neben der sehr guten oberflächenaktiven Wirkung zeichnen sich diese Produkte insbesondere durch ihre problemlose biologische Abbaubarkeit aus. Aufgrund ihrer guten Hautverträglichkeit finden sie bspw. im Kosmetikbereich vielfach Anwendung.

Bei der Herstellung von Zuckerestern werden die entsprechenden Kohlenhydrate, z.B. Glucose oder Saccharose, mit acylierenden Reagenzien, wie Fettsäurederivaten (Fettsäuremethylestern oder Glyceriden), über Veresterungs- bzw. Umesterungsschritte bis zum Erreichen des gewünschten Acylierungsgrades umgesetzt. Die Acylierung verläuft jedoch nicht einheitlich und so bestehen die Reaktionsprodukte aus mono-, di- und mehrfach acylierten Zuckern sowie wechselnden Anteilen des nicht umgesetzten Ausgangsmaterials.

Oberflächenaktive Alkylglycoside werden meist direkt durch Reaktion von Zuckern mit Fettalkoholen erhalten, wobei das Produktgemisch jedoch noch größere Mengen an nicht umgesetztem Fettalkohol enthält.

Obwohl die oben genannten Reaktionsprodukte bereits eine gute oberflächenaktive Wirkung zeigen, sind für bestimmte Anwendungsbereiche weitere Aufreinigungsmaßnahmen unumgänglich, die die Abtrennung unerwünschter Nebenprodukte, wie z.B. nicht umgesetzter Fettsäuren, Fettsäuremethylester, Glyceride oder Fettalkohole, zum Ziel haben.

Aus dem Stand der Technik sind hierfür u.a. destillative Verfahren und konventionelle Lösemittelverfahren bekannt, die jedoch mit erheblichen Nachteilen behaftet sind. Bei destillativen Verfahren ist die relativ hohe Temperaturempfindlichkeit der gewünschten Produkte zu beachten. Generell neigen Kohlenhydratderivate leicht zur Karamellisierung, wobei sie sich dunkel färben. Ferner besteht bei einer thermischen Belastung auch die Tendenz zur Disproportionierung, so daß sich während des Destillationsvorganges die Zusammensetzung der Reaktionsprodukte verändert. Außerdem neigen die Produkte bei der Destillation oft zum unkontrollierten Schäumen. Lösemittelverfahren haben wiederum den Nachteil, daß die eigentlich gewünschte oberflächenaktive Wirkung der Zielprodukte eine effiziente Trennung der Produktgemische technisch sehr erschwert, da im Reaktionsmedium eine Emulsionsbildung auftritt.

Andererseits sind aus der Literatur zahlreiche Verfahren bekannt, die die Extraktion lipophiler Substanzen mit verdichteten Gasen beschreiben. Vor allem verdichtete Kohlenwasserstoffe werden bereits seit längerem als Medien zur Extraktion von Fetten und Ölen verwendet.

So ist aus der US-Patentschrift 2 548 434 ein selektives Verfahren zur Extraktion und Fraktionierung von fettartigen Materialien bekannt, bei dem mit Hilfe von verflüssigten Kohlenwasserstoffen wie Propan, Butan u.a. oder Mischungen davon bei Temperaturen zwischen 66 und 93,3°C aus Getreideschrot, Schwarten und Bleicherde farblose Fette erhalten und von den unerwünschten, dunkelgefärbten Bestandteilen abgetrennt werden.

Ein weiteres Verfahren zur Flüssigextraktion von Öl enthaltenden Materialien wie Ölsaaten, Schwarten, Fischmehl u.a. mit Hilfe von verflüssigten Kohlenwasserstoffen wie Propan beschreibt die US-Patentschrift 2 682 551.

Die Reinigung von Kohlenhydraten durch Extraktion mit verdichteten Gasen bzw. Gasgemischen ist bisher nicht bekannt. Insbesondere ist keines der beschriebenen Verfahren dazu geeignet, synthetische Kohlenhydratderivate problemlos und ohne Zuhilfenahme mehrerer Verfahrensstufen im wirtschaftlichen Rahmen von den unerwünschten Nebenprodukten zu reinigen.

Ziel der vorliegenden Erfindung war es somit, ein Verfahren zur Reinigung von Kohlenhydratderivaten mit oberflächenaktiver Wirkung zu entwickeln, das die Nachteile der oben genannten Verfahrensweisen des Standes der Technik nicht aufweist, sondern es ermöglicht, auf produktschonende und wirtschaftliche Art Kohlenhydratderivate von hoher Reinheit bereitzustellen.

Gelöst wurde diese Aufgabe erfindungsgemäß dadurch, daß ein die Kohlenhydratderivate und Verunreinigungen enthaltendes Ausgangsmaterial mit verdichtetem Propan oder dessen Mischungen mit bis zu 25 Gew-% Butan bei Drücken zwischen 8 und 150 bar, insbesondere zwischen 20 und 80 bar, und Temperaturen < 120°C extrahiert wird, wobei die Kohlenhydratderivate von Verunreinigungen abgetrennt werden.

Es hat sich nämlich überraschend gezeigt, daß sich verdichtetes Propan und dessen Mischungen mit Butan im flüssigen und nahe kritischen Zustand unerwartet gut dazu eignen, Reaktionsprodukte, wie z.B. Fettsäuren, Fettsäureester, Glyceride oder Fettalkohole, aus einem Reaktionsgemisch, das bei der Herstellung von Zuckerestern oder Alkylglycosiden entsteht, abzutrennen. Dabei wurde gefunden, daß die im Reaktionsgemisch enthaltenen Verunreinigungen eine höhere Löslichkeit als die Kohlenhydratderivate aufweisen.

Die Extraktion kann abhängig vom festen oder fließfähigen bis flüssigen Aggregatzustand des Ausgangsmaterials in zwei Verfahrensweisen durchgeführt werden. Während feste Ausgangsmaterialien im allgemeinen batchweise nach dem Prinzip einer Fest/Flüssig-Extraktion gereinigt werden, kann bei fließfähigen bis flüssigen Ausgangsmaterialien eine kontinuierliche Flüssig/Flüssig-Extraktion erfolgen; dann wird das Verfahren vorzugsweise in einer Trennkolonne, vorzugsweise im Gegenstromprinzip, durchgeführt. Das Extraktionsmedium wird dabei dem Stoffgemisch des zu trennenden Ausgangsmaterials entgegen, geführt, wobei sich die Zuckerderivate (Ester, Alkylglycoside) im Extraktionsmedium nicht oder nur sehr schlecht lösen und sich im Sumpfprodukt der Kolonne anreichern. Die abzutrennenden Verunreinigungen bzw. Nebenkomponenten, z.B. Fettsäuren, Fettsäureester, Glyceride oder Fettalkohole, sind im verdichteten Gas(-gemisch) unter den erfindungsgemäßen Bedingungen sehr gut löslich und werden mit dem Extraktionsmedium als sogenannte Kopfprodukte aus der Kolonne abgeführt. Dabei kann die Trennung in der Kolonne hinsichtlich der Trennschärfe verbessert werden, indem die als Trennkolonne fungierende Extraktionskolonne mit einem Temperaturgradienten ΔT ≤ 20°C beheizt wird, wobei die höhere Temperatur ausnahmslos im Kopf der Kolonne eingestellt wird und als Temperaturobergrenze im Kopf die maximale Verfahrenstemperatur von 120°C gilt.

Prinzipiell sind auch höhere als die erfindungsgemäßen Verfahrenstemperaturen möglich, jedoch sind diese aufgrund der beschriebenen Temperaturempfindlichkeit der Produkte nicht zu empfehlen, da Zersetzungsreaktionen auftreten. Die untere Grenze der Verfahrenstemperatur geben der jeweilige Verfahrensdruck sowie ganz allgemein Aspekte der Wirtschaftlichkeit vor.

Abhängig vom zu trennenden Stoffgemisch besitzen die verwendeten verdichteten Gase oder deren Mischungen ein stets sehr gutes Lösevermögen für die abzutrennden Verunreinigungen, so daß sich das Verfahren durch sehr gute Raum-Zeit-Ausbeuten auszeichnet. Typischerweise werden beim beschriebenen Verfahren pro Gramm zu trennendes Stoffgemisch 3 bis 15 Gramm an verdichtetem Gas (-gemisch) benötigt.

Ein überraschender Vorteil des erfindungsgemäßen Verfahrens ist auch darin zu sehen, daß sich allgemein das verdichtete Extraktionsgas(-gemisch) zumindest in Anteilen im zu trennenden Stoffgemisch löst und dadurch speziell bei den fließfähigen bis flüssigen Ausgangsstoffen deren sonst teilweise hohen Viskositäten erniedrigt. Aufgrund dieser Viskositätserniedrigung wird bei den genannten Stoffgemischen eine effektive Extraktion erst möglich, da der limitierende Stoffübergang an der Phasengrenzfläche erheblich verbessert wird.

Die Abtrennung der gereinigten Produkte vom Extraktionsmedium ist ebenfalls unproblematisch und kann z.B. durch Druckabsenkung und/oder Temperaturerhöhung erreicht werden, wobei das Extraktionsgas(-gemisch) in den gasförmigen Zustand überführt wird, so daß ein Phasenübergang vom flüssigen bzw. nahe kritischen in den gasförmigen Zustand des Extraktionsmediums erfolgt.

Das Extraktionsmedium kann anschließend durch Rekompression und/oder Verflüssigung zurückgewonnen und erneut in den Extraktionsprozeß zurückgeführt werden.

Besonders vorteilhaft ist das Verfahren, wenn die gereinigten Kohlenhydratderivate als feste Produkte anfallen. Dadurch, daß sich das verdichtete Gas(-gemisch) unter Druck im Produkt löst und dieses dadurch in einer Schmelze hält, kann das Produkt aus dem Kolonnensumpf durch eine geeignete Düsenvorrichtung ausgetragen werden, wobei einerseits das Gas(-gemisch) nach Austritt spontan verdampft und andererseits das reine Produkt als feines Pulver anfällt.

Folgende Beispiele sollen das erfindungsgemäße Verfahren verdeutlichen.

### Beispiel 1

### Reinigung eines Gemisches aus Methylglycosidestern der Öl-, Stearin-, Linol- und Palmitinsäure von den entsprechenden freien Fettsäuren

Ein Reaktionsgemisch aus der Herstellung von Methylglycosidestern der Öl-, Stearin-, Linol- und Palmitinsäure (in Summe der Monoester und höheren Ester zusammen 79 Gew.-%) war mit 16 Gew.-% der entsprechenden freien Fettsäuren verunreinigt. Das bei 55 °C aufgeschmolzene Ausgangsmaterial wurde in einer Hochdruckextraktionskolonne (Kolonnenvolumen 2 l, Höhe 2 m) in einem Anteil von 10 Gew.-% dem aufsteigenden verdichteten Propan bei 50 bar und 85 °C im Gegenstrom zugeführt. Aufgrund der schlechteren Löslichkeit reichern sich die polaren Kohlenhydratderivate vorwiegend im Kolonnensumpf an, während die freien Fettsäuren mit dem Extraktionsmedium über den Kolonnenkopf geführt werden. Das überwiegend die freien Fettsäuren enthaltende Kopfprodukt wurde einem Extraktabscheider zugeführt, in dem durch Verdampfung des flüssigen Propans bei 10 bar und 60 °C die extrahierten Verbindungen ausfielen. Das Extraktionsgas wurde anschließend wieder verflüssigt und erneut in den Extraktionsprozeß eingespeist. Das Sumpfprodukt (80 Gew.-% bezogen auf die eingespeiste Menge) wurde direkt aus der Kolonne über eine geeignete Austragsvorrichtung auf Atmosphärendruck entspannt, wobei die gereinigten Methylglucosidester anfielen (in Summe der Monoester und höheren Ester zusammen 91 Gew.-%, freien Fettsäuren 6 Gew.-%).

### Beispiel 2

### Reinigung eines Gemisches aus Methylglycosidestern der Isostearinsäure von der entsprechenden freien Isostearinsäure

Ein Reaktionsgemisch aus der Herstellung von Methylglycosidestern der Isostearinsäure (in Summe der Monoester und höheren Ester zusammen 71 Gew.-%) war mit 21 Gew.-% der entsprechenden freien Fettsäure verunreinigt. Das bei 55 °C aufgeschmolzene Ausgangsmaterial wurde in einer Hochdruckextraktionskolonne (Kolonnenvolumen 2 l, Höhe 2 m) in einem Anteil von 15 Gew.-% dem aufsteigenden verdichteten Propan bei 80 bar und 95 °C im Gegenstrom zugeführt. Aufgrund der schlechteren Löslichkeit reichern sich die polaren Kohlenhydratderivate vorwiegend im Kolonnensumpf an, während die freien Fettsäuren mit dem Extraktionsmedium über den Kolonnenkopf geführt werden. Das überwiegend die freien Fettsäuren enthaltende Kopfprodukt wurde einem Extraktabscheider zugeführt, in dem durch Verdampfung des flüssigen Propans bei 10 bar und 60 °C die extrahierten Verbindungen ausfielen. Das Extraktionsgas wurde anschließend wieder verflüssigt und erneut in den Extraktionsprozeß eingespeist. Das Sumpfprodukt (73 Gew.-% bezogen auf die eingespeiste Menge) wurde direkt aus der Kolonne über eine geeignete Austragsvorrichtung auf Atmosphärendruck entspannt, wobei die gereinigten Methylglucosidester anfielen (in Summe der Monoester und höheren Ester zusammen 85 Gew.-%, freien Fettsäuren 6 Gew.-%).

### Beispiel 3

### Reinigung eines Gemisches aus Alkylpolyglycosiden (Mono-, Di-, Trisaccharide) und entsprechenden freien Fettalkoholen der Kettenlänge C₁₂ bis C₁₈

Ein Reaktionsgemisch aus der Herstellung von Alkylpolyglycosiden (Mischung aus Mono-, Di-, Trisacchariden, 25 Gew.-%) war mit 70 Gew.-% der entsprechenden freien Fettalkohole der Kettenlänge C₁₂ bis C₁₈ verunreinigt. Das aufgeschmolzene Ausgangsmaterial wurde in einer Hochdruckextraktionskolonne (Kolonnenvolumen 2 l, Höhe 2 m) in einem Anteil von 15 Gew.-% dem aufsteigenden verdichteten Propan bei 80 bar und 85 °C im Gegenstrom zugeführt. Aufgrund der schlechteren Löslichkeit reichern sich die polaren Kohlenhydratderivate vorwiegend im Kolonnensumpf an, während die Fettalkohole mit dem Extraktionsmedium über den Kolonnenkopf geführt werden. Das überwiegend die Fettalkohole enthaltende Kopfprodukt wurde einem Extraktabscheider zugeführt, in dem durch Verdampfung des flüssigen Propans bei 10 bar und 60 °C die extrahierten Verbindungen ausfielen. Das Extraktionsgas wurde anschließend wieder verflüssigt und erneut in den Extraktionsprozeß eingespeist. Das Sumpfprodukt (20 Gew.-% bezogen auf die eingespeiste Menge) wurde direkt aus der Kolonne über eine geeignete Austragsvorrichtung auf Atmosphärendruck entspannt, wobei die gereinigten Alkylpolyglycoside pulverförmig anfielen (Reinheit > 90 Gew.-%, Fettalkohole < 5 Gew.-%).

## Patentansprüche

1. Verfahren zur Reinigung von Kohlenhydratderivaten mit oberflächenaktiver Wirkung mit Hilfe von verdichteten Gasen, **dadurch gekennzeichnet,**daß ein die Kohlenhydratderivate und Verunreinigungen enthaltendes Ausgangsmaterial mit verdichtetem Propan oder dessen Mischungen mit bis zu 25 Gew.-% Butan bei Drücken zwischen 8 und 150 bar und Temperaturen < 120°C extrahiert wird, wobei die Kohlenhydratderivate von Verunreinigungen abgetrennt werden.

2. Verfahren nach Anspruch 1 **dadurch gekennzeichnet,** daß bei Drücken zwischen 20 und 80 bar extrahiert wird.

3. Verfahren nach einem der Ansprüche 1 oder 2, dadurch gekennzeichnet, daß die Verunreinigungen eine höhere Löslichkeit im Extraktionsmedium als die Kohlenhydratderivate besitzen.

4. Verfahren nach einem der Ansprüche 1 bis 3, dadurch gekennzeichnet, daß feste Ausgangsmaterialien batchweise einer Fest/Flüssig-Extraktion unterzogen werden.

5. Verfahren nach Anspruch 1 bis 3, **dadurch gekennzeichnet,** daß fließfähige bis flüssige Ausgangsmaterialien einer Flüssig/Flüssig-Extraktion unterzogen werden.

6. Verfahren nach Anspruch 5, **dadurch gekennzeichnet,** daß man die Extraktion im Gegenstrom durchführt.

7. Verfahren nach einem der Ansprüche 5 oder 6, **dadurch gekennzeichnet,** daß die Extraktion in einer Trennkolonne mit einem zum Kopf hin ansteigenden Temperaturgradienten mit ΔT ≤ 20°C durchgeführt wird.

8. Verfahren nach einem der Ansprüche 1 bis 7, **dadurch gekennzeichnet,** daß pro Gramm zu extrahierendem Ausgangsmaterial 3 bis 15 Gramm verdichtetes Gas(-gemisch) eingesetzt werden.

9. Verfahren nach einem der Ansprüche 1 bis 8, **dadurch gekennzeichnet,** daß die Gewinnung der Kohlenhydratderivate durch Druckabsenkung und/oder Temperaturerhöhung erfolgt, wobei das Gas(-gemisch) in den gasförmigen Zustand überführt wird.

10. Verfahren nach einem der Ansprüche 1 bis 9, **dadurch gekennzeichnet,** daß das Extraktionsmedium im Kreislauf geführt wird.

11. Verfahren nach einem der Ansprüche 1-10, **dadurch gekennzeichnet,** daß die Kohlenhydratderivate aus mono-, di- oder/und oligosaccharidischen Kohlenhydraten mit lipophilen Gruppen, insbesondere Kohlenhydratestern, Kohlenhydratestern, veresterten Kohlenhydratethern oder Gemischen davon, ausgewählt werden.

12. Verfahren nach einem der Ansprüche 1-11, **dadurch gekennzeichnet,** daß als Verunreinigungen lipophile Substanzen, insbesondere Fettsäuren, Fettsäureester, Glyceride oder/und Fettalkohole, abgetrennt werden.

## Claims

1. Process for the purification of carbohydrate derivatives with a surface-active action with the aid of compressed gases, **wherein** a starting material containing the carbohydrate derivatives and impurities is extracted with compressed propane or mixtures thereof containing up to 25 % by weight butane at pressures between 8 and 150 bar and at temperatures of < 120°C in the process of which the impurities are separated from the carbohydrate derivatives.

2. Process as claimed in claim 1, **wherein** the extraction is carried out at pressures between 20 and 80 bar.

3. Process as claimed in one of the claims 1 or 2, **wherein** the impurities have a higher solubility in the extraction medium than the carbohydrate derivatives.

4. Process as claimed in one of the claims 1 to 3, **wherein** solid starting materials are subjected batch-wise to a solid/liquid extraction.

5. Process as claimed in claims 1 to 3, **wherein** flowable to liquid starting materials are subjected to a liquid/liquid extraction.

6. Process as claimed in claim 5, **wherein** the extraction is carried out in a countercurrent.

7. Process as claimed in one of the claims 5 or 6, **wherein** the extraction is carried out in a separation column with a temperature gradient of ΔT ≤ 20°C that increases towards the top.

8. Process as claimed in one of the claims 1 to 7, **wherein** 3 to 15 gram compressed gas (mixture) is used per gram of starting material to be extracted.

9. Process as claimed in one of the claims 1 to 8, **wherein** the carbohydrate derivatives are isolated by reducing the pressure and/or increasing the temperature during which the gas (mixture) is converted into a gaseous state.

10. Process as claimed in one of the claims 1 to 9, **wherein** the extraction medium is recycled.

11. Process as claimed in one of the claims 1 to 10, **wherein** the carbohydrate derivatives are selected from mono-, di- or/and oligosaccharidic carbohydrates having lipophilic groups in particular carbohydrate esters, carbohydrate ethers, esterified carbohydrate ethers or mixtures thereof.

12. Process as claimed in one of the claims 1 to 11, **wherein** lipophilic substances in particular fatty acids, fatty acid esters, glycerides or/and fatty alcohols are separated as impurities.

## Revendications

1. Procédé pour la purification de dérivés d'hydrates de carbone doués d'une activité tensioactive à l'aide de gaz comprimés, caractérisé en ce qu'une matière de départ contenant les dérivés d'hydrates de carbone et des impuretés est extraite avec du propane comprimé ou ses mélanges avec jusqu'à 25% en poids de butane sous des pressions entre 8 et 150 bars et à des températures < 120°C, moyennant quoi les dérivés d'hydrates de carbone sont séparés des impuretés.

2. Procédé selon la revendication 1, caractérisé en ce qu'on extrait à des pressions entre 20 et 80 bars.

3. Procédé selon l'une des revendications 1 ou 2, caractérisé en ce que les impuretés possèdent une solubilité plus élevée dans le milieu d'extraction que les dérivés d'hydrates de carbone.

4. Procédé selon l'une des revendications 1 à 3, caractérisé en ce que des matières de départ solides sont soumises à une extraction solide/liquide discontinue.

5. Procédé selon l'une des revendications 1 à 3, caractérisé en ce que des matières de départ fluides à liquides sont soumises à une extraction liquide/liquide.

6. Procédé selon la revendication 5, caractérisé en ce qu'on effectue l'extraction à contre-courant.

7. Procédé selon l'une des revendications 5 ou 6, caractérisé en ce que l'extraction est conduite dans une colonne de séparation avec un gradient de température croissant vers le sommet avec ΔT ≤ 20°C.

8. Procédé selon l'une des revendications 1 à 7, caractérisé en ce qu'on met en oeuvre 3 à 15 grammes de gaz (ou mélange de gaz) comprimé par gramme de matière de départ à extraire.

9. Procédé selon l'une des revendications 1 à 8, caractérisé en ce que l'obtention des dérivés d'hydrates de carbone a lieu par abaissement de la pression et/ou augmentation de la température, auquel cas le gaz (ou mélange de gaz) est amené à l'état gazeux.

10. Procédé selon l'une des revendications 1 à 9, caractérisé en ce que le milieu d'extraction est recyclé.

11. Procédé selon l'une des revendications 1-10, caractérisé en ce que les dérivés d'hydrates de carbone sont choisis parmi des hydrates de carbone mono-, di- ou/et oligosaccharidiques avec des groupes lipophiles, notamment des esters d'hydrates de carbone, des éthers d'hydrate de carbone, des éthers d'hydrates de carbone estérifiés ou leurs mélanges.

12. Procédé selon l'une des revendications 1-11, caractérisé en ce que des substances lipophiles, en particulier des acides gras, des esters d'acides gras, des glycérides ou/et des alcools gras sont séparés en tant qu'impuretés.
